# FASCICULE DE BREVET EUROPEEN

(11) **EP 1 507 517 B1**
(45) Date de publication et mention de la délivrance du brevet: **08.03.2017**
(21) Numéro de dépôt: 03747486.3
(22) Date de dépôt: 02.05.2003
(51) Int. Cl.: A61K 9/16, A61K 47/00, A61L 27/38, A61K 9/50, A61L 27/18, A61L 27/54, A61L 27/58

(54) **MICROPARTICULES SUPPORTANT DES CELLULES ET DES SUBSTANCES ACTIVES**
MIKROPARTIKEL MIT ZELLEN UND WIRKSTOFFEN
MICROPARTICLES BEARING CELLS AND ACTIVE SUBSTANCES

(30) Priorité: 03.05.2002 FR 0205574
(43) Date de publication de la demande: 23.02.2005
(73) Titulaire: INSERM, 75654 Paris Cédex 13 (FR)
(72) Inventeur: MONTERO-MENEI, Claudia, 49100 Angers (FR); MENEI, Philippe, 49100 Angers (FR); BENOIT, Jean-Pierre, 49100 Angers (FR); TATARD, Valérie, 49100 Angers (FR); VENIER, Maire-Claire, 49610 Juigne-sur-Loire (FR)
(74) Mandataire: Cabinet Plasseraud
(86) Numéro de dépôt international: PCT/FR2003/001377
(87) Numéro de publication internationale: WO 2003/092657

(56) Documents cités:
- EP-A- 0 030 885
- WO-A-90/10018
- WO-A1-93/23088
- WO-A1-98/28357
- WO-A2-01/70290
- WO-A2-98/51812
- US-A- 5 846 565
- US-B1- 6 309 669
- US-B1- 6 376 644
- DATABASE WPI Section Ch, Week 199304 Derwent Publications Ltd., London, GB; Class A96, AN 1993-032611 XP002225546 & JP 04 360682 A (KURABO IND LTD), 14 décembre 1992 (1992-12-14)
- DATABASE WPI Section Ch, Week 199306 Derwent Publications Ltd., London, GB; Class B04, AN 1993-049148 XP002225547 & JP 05 000081 A (KOKEN KK), 8 janvier 1993 (1993-01-08)
- DATABASE WPI Section Ch, Week 199749 Derwent Publications Ltd., London, GB; Class A23, AN 1997-532839 XP002225548 & JP 09 255777 A (NIPPON GOSEI GOMU KK), 30 septembre 1997 (1997-09-30)
- Melissa J Mahoney ET AL: "Transplantation of brain cells assembled around a programmable synthetic microenvironment", Nature Biotechnology, vol. 19, no. 10 1 October 2001 (2001-10-01), pages 934-939, XP055268379, United States DOI: 10.1038/nbt1001-934 Retrieved from the Internet: URL:http://www.nature.com/nbt/journal/v19/ n10/pdf/nbt1001-934.pdf [retrieved on 2016-04-25]

## Description

La présente invention se rapporte au domaine de la préparation et de la transplantation de cellules utiles dans le cadre de thérapie cellulaire pour la réparation tissulaire ou pour le transfert de gène, ou encore pour la vaccination. Plus particulièrement, l'invention à pour objet des microparticules à base d'un matériau biocompatible et biodégradable, comportant des cellules d'intérêts et des facteurs de croissance ou de cytokines.

La thérapie cellulaire, par greffe de cellules autologues ou non autologues, constitue un outil thérapeutique majeur qui est aujourd'hui essentiellement développé en hématobiologie, mais devrait s'appliquer à d'autres spécialités, du fait des connaissances acquises sur les cellules souches et leur identification dans la plupart des tissus, du muscle au système nerveux central. L'identification et la caractérisation croissantes de cytokines et de facteurs de croissance permettent d'envisager la possibilité de contrôler *in vitro* et/ou in vivo la prolifération et la différenciation de ces cellules, et de moduler leur environnement tissulaire (phénomènes immunologiques de rejet, angiogénèse). Malgré ces avancées en biologie cellulaire, le développement clinique des greffes de cellules reste actuellement limité, en raison notamment du faible taux de survie des cellules implantées, qui peut être lié à une mortalité non spécifique (mort cellulaire par nécrose ou apoptose) due aux procédures de recueil, de conservation, de transformation et d'administration ou à un rejet immunologique (dans les allo et xénogreffes), voire à l'absence d'intégration dans le tissu hôte.

Afin de réduire cette mortalité cellulaire, on a proposé l'utilisation de microbilles non biodégradables sur lesquelles adhèrent les cellules, servant ainsi de transporteurs ou « microcarriers ». La survie et le fonctionnement d'hépatocytes sont par exemple améliorés quand ces cellules sont greffées, adhérentes à des microbilles de verre ou de dextrane (Cytodex^{R}) (Demetriou et al., 1986 ; Te Velde et al., 1992). Cette stratégie a permis d'obtenir des résultats plus intéressants qu'avec des hépatocytes microencapsulés. Plus récemment, ces mêmes microbilles ont été utilisées pour cultiver et greffer des kératinocytes humains afin de reconstituer une couverture cutanée chez la souris nude (Voigt et al., 1999). Cette approche a aussi été utilisée pour greffer des cellules neuro-chromaffines ou des neurones embryonnaires dopaminergiques, dans un modèle murin de la maladie de Parkinson. Dans ce modèle, la survie des cellules transplantées dans le striatum est fortement augmentée quand elles sont préalablement adhérées aux microparticules de verre ou de dextran, et permettent une amélioration comportementale des animaux (Cherskey et al., 1996 ; Saporta et al., 1997 ; Borlongan et al., 1998). De même, il a été observé (Saporta et al., 1997) que des cellules foetales humaines adhérées sur des microbilles de dextrane survivent au moins trois mois sans traitement immunodépresseurs, alors que sans microparticules ces cellules sont rejetées rapidement.

Une autre approche, plus récente, permettant d'augmenter la survie de cellules greffées est l'administration, en association avec la greffe, de facteurs de croissance. Ces protéines qui peuvent agir sur la prolifération, la différenciation, l'activation, et la survie des cellules, constituent un apport majeur en greffe cellulaire. Bien qu'il soit maintenant possible de disposer de facteurs de croissance recombinants humains, leur administration présente une difficulté car ces protéines ont une demi-vie courte et traversent mal certaines barrières biologiques. Elles ont de plus une action pléïotropique, pouvant être la cause d'effets secondaires indésirables. Aucun des modes d'administration actuellement développé n'est totalement satisfaisant et applicable en clinique rapidement.

Un des premiers modes d'administration proposé consistait à greffer les cellules dans une suspension contenant le facteur de croissance. Cette approche, si elle est simple, ne permet pas d'agir à long terme sur les cellules. Un deuxième mode d'administration consiste à co-transplanter un tissu identifié pour produire le facteur de croissance choisi, par exemple les co-greffes nerf périphérique-cellules chromaffines (Date et al., 1996), ou les co-greffes hépatocytes-ilôts de Langherans (Kneser et al., 1999). La survie parfois limitée de ces co-greffes et l'impossibilité de contrôler les doses de facteurs de croissance limitent considérablement cette stratégie. Les progrès de la biologie moléculaire permettent maintenant d'obtenir des cellules génétiquement modifiées produisant un facteur de croissance, et qui peuvent être utilisées en co-greffes ou comme greffe proprement dite (Menei et al 1998 ; Wood et Prior, 2001). Cette approche reste cependant limitée par des problèmes éthiques, des risques biologiques, et le contrôle des doses libérées. Ainsi, il a été rapporté des greffes de cellules nerveuses, comme des cellules neuro-endocriniennes PC12 (Menei et al., 1989 ; Dehaut et al., 1993) ou cellules de Schwann normales ou génétiquement modifiées pour produire un facteur neurotrophique (Montero-Menei et al., 1992 ; Menei et al., 1998).

Il a également été décrit des microparticules biodégradables libérant des molécules neuroactives, de façon contrôlée et prolongée (Menei et al., 1997 ; Benoit et al., 1999). Ces microsphères sont constituées d'un biopolymère de type poly(acide lactique-co-acide glycolique) (PLGA). Elles sont biocompatibles avec le tissu nerveux et totalement dégradées en quelques mois (Menei et al., 1993 ; 1994b ; Véziers et al., 2000). Leur taille de quelques dizaines de microns permet une implantation stéréotaxique dans le cerveau, au niveau de leur cible pharmacologique, en utilisant les mêmes micro-seringues que pour les implantations de cellules (Menei et al., 1994a). Elles ont été utilisées avec succès dans une étude clinique de phase I pour la chimiothérapie interstitielle des tumeurs cérébrales (Menei et al., 1999).

Des microsphères libérant des protéines, en particulier les facteurs de croissance et les cytokines ont également été développées. Le« nerve growth factor » (NGF), est une substance intéressante car parmi les plus anciennement caractérisée. Ainsi, il a été décrit des microsphères pouvant libérer du NGF pendant au moins deux mois (Péan et al., 1998 ; Péan et al., 1999). Leur intérêt thérapeutique a été démontré sur deux modèles animaux de maladies neurodégénératives : modèle murin de la maladie d'Alzheimer, (Péan et al., 2000) et le modèle murin de la Chorée de Huntington (Menei et al., 2000).

Dans le domaine des tumeurs, il a été formulé des microsphères de PLGA capables de libérer des cytokines immunostimulantes après implantation intra-tumorale (Mullerad et al. 2000, Pettit et al., 1997). L'utilisation de microsphères biodégradables pour la libération des cytokines dans le cadre de vaccin anti-tumoral a ainsi été proposée (Golumbek et al, 1993), mais, dans cette étude, les microsphères sont simplement mélangées aux cellules immédiatement avant injection. De même, la préparation de vaccin constitué de microsphères recouvertes d'antigènes bactériens ou de vésicules membranaires a été déjà proposée, mais sans que la microsphère puisse libérer une molécule immunostimulante (Mescher et Rogers, 1996, Mesher et Savelieva, 1997).
WO9323088 décrit des microparticules avec des cellules de peau à la surface pour le traitement de blessures cutanées.

Le but de la présente invention est d'offrir maintenant une combinaison de cellules (ou de fractions de cellules) et de substances actives sur ces cellules, comme des facteurs de croissance ou des cytokines, au niveau des mêmes microparticules pour la greffe de cellules en thérapie cellulaire ou la vaccination.

Les compétences des inventeurs leur ont permis de développer des microcarriers pharmacologiquement actifs, aussi désignés ci-après « MPA » qui libèrent des facteurs de croissance de façon prolongée et contrôlée. Ces microparticules de quelques dizaines de microns de diamètre sont constituées de polymères biodégradables et biocompatibles permettant l'adhérence des cellules ou de fragments cellulaires grâce aux propriétés propres du polymère ou à un enrobage qui peut être biologiquement actif. Les MPA ne présentent pas de risque biologique et sont remarquables car :
- Ils peuvent servir de support pour la culture des cellules. L'adhésion préférentielle des cellules à greffer sur les microparticules permet leur préparation voire leur transformation *in vitro* sans utiliser pour les recueillir, d'enzymes protéolytiques d'origine animale déconseillées pour des raisons de sécurité sanitaires évidentes.
- Ils peuvent servir de support aux cellules greffées et se dégrader sans toxicité après l'implantation, n'interférant pas avec l'intégration des cellules greffées.
- Ils peuvent libérer un ou plusieurs facteurs de croissance ou cytokines pendant une durée programmée et à une dose déterminée.
- Ils peuvent promouvoir la survie et la différenciation des cellules greffées, modifier leur microenvironnement, ainsi que leur intégration dans le tissu hôte.

L'invention a donc pour objet des microparticules à base d'un matériau biocompatible et biodégradable, caractérisées en ce qu'elles comportent à leur surface des cellules d'intérêt et en ce qu'elles comprennent des molécules d'au moins une substance active sur lesdites cellules ou leur environnement lors de l'implantation des microparticules, lesdites molécules étant libérées par les microparticules de façon prolongée et contrôlée.

L'invention a plus précisément pour objet des microparticules à base d'un matériau biocompatible et biodégradable,

lesdites particules comportant à leur surface des cellules d'intérêt choisies parmi des cellules embryonnaires ou des cellules souches,

lesdites particules comprenant des molécules d'au moins une substance active sur lesdites cellules ou leur environnement lors de l'implantation des microparticules et choisie parmi les facteurs de croissance, les cytokines, les immunomodulateurs ou facteurs agissant sur la différenciation cellulaire, lesdites molécules étant libérées par les microparticules de façon prolongée et contrôlée,

lesdites microparticules étant enrobées d'un composé permettant l'adhésion des cellules, ledit composé étant en outre éventuellement actif sur lesdites cellules, et choisi parmi la poly-D-lysine, la poly-L-lysine, la polyornithine, la polyéthylèneamine, ou molécule synthétique ou non appartenant à la matrice extracellulaire choisie parmi la fibronectin-like, des polymères synthétiques sur lesquels sont greffées des séquences RGD ou de la lysine ou un mélange de ceux-ci,

lesdites microparticules ayant un diamètre compris entre 10 et 500 µm, et ledit matériau biodégradable et biocompatible étant un polymère ou un copolymère de polyester.

L'invention admet plusieurs modes de réalisation selon que les molécules d'au moins une substance active sont à la surface et/ou incorporées dans les microparticules.

L'incorporation de la molécule active peut être réalisée lors du processus d'encapsulation et/ou après la formation des particules. Selon les conditions opératoires, la matrice peut-être plus ou moins poreuse avec une forme plus ou moins sphérique.
Les microparticules sont constituées d'un polymère ou d'un copolymère de polyester biocompatible et biodégradable. Un tel polymère ou copolymère est par exemple choisi dans le groupe comprenant les poly(α-hydroxyacides), comme les polylactides, les polylactides co-glycolides, les poly ε-coprolactones, le PLGA, et leur mélange. Dans un mode de réalisation avantageux de l'invention, le polymère est choisi parmi les polylactides Les microparticules présentent un diamètre compris entre 10 et 500 µm. En effet, les MPA présentent l'avantage de pouvoir adapter la taille des microparticules en fonction des cellules adhérées.

L'adhésion des cellules sur les microparticules est permise par les propriétés propres du polymère et par un enrobage avec un composé ou mélange de composés permettant l'adhésion des cellules et pouvant être biologiquement actif(s). Ainsi, l'invention envisage l'utilisation de polymère synthétique permettant l'adhésion cellulaire par leurs propriétés physico-chimiques, ou des copolymères synthétiques sur les molécules desquelles sont greffées des séquences RGD ou de la lysine (Varani et al. 1993). Les composés d'enrobage des microparticules permettant l'adhésion des cellules sont choisis dans le groupe comprenant la poly-D-lysine, la poly-L-lysine, la polyornithine, la polyethyleneamine, ou tout autre molécule synthétique ou non appartenant à la matrice extracellulaire comme la fibronectin-like, ou encore le mélange de ceux-ci.

Ces microparticules comprennent des molécules d'au moins une substance active sur les cellules ou l'environnement de ces cellules lors de greffe. Des procédés d'encapsulation de ces molécules sont divers, et l'on peut citer un procédé par double émulsion, ou tout autre procédé physicochimique, mécanique ou chimique. Il peut aussi s'agir d'une simple imbibition des microparticules avec lesdites molécules de façon à les fixer à la surface des microparticules. Les microparticules libèrent des molécules de cette substance, de façon prolongée et contrôlée.

Selon une première forme de mise en oeuvre de l'invention, les MPA sont utiles pour la préparation de compositions pharmaceutiques utiles en thérapie cellulaire pour la réparation tissulaire ou pour le transfert de gène.

Les cellules d'intérêt fixées à la surface des microparticules peuvent être diverses selon le type de greffe désiré, il peut s'agir de cellules embryonnaires, ou des cellules souches. Ces cellules peuvent être utilisées dans différentes pathologies nécessitant une thérapie cellulaire. C'est par exemple le cas des greffes d'hépatocytes et des îlots de Langherans. Dans le cas de neurotransplantation, il peut s'agir d'une lignée cellulaire PC12 capables de secréter de la dopamine et de se différencier en neurones « sympathiques-like » sous l'effet du NGF.

Les molécules des substances incorporées ou à la surface des MPA sont les facteurs de croissance, les cytokines, les immunomodulateurs ou facteurs agissant sur la différenciation cellulaire, notamment ceux choisis dans le groupe comprenant les neutrophines comme le NGF, le BNDF, le NT-3, etc..., les TGFβs, la famille du GDNF, les FGFs, l'EGF, le PDGF, les interleukines comme l'Il-1, l'Il-2, les chemokines, l'acide rétinoïque, l'erythropoïtine, etc., ou mélange de ceux-ci.

Les molécules libérées par les microparticules seules ou en combinaison avec le composé d'enrobage pour l'adhésion des cellules favorisent la survie des cellules, leur fonction ou orientent la différenciation des cellules souches vers un phénotype déterminé. Elles peuvent aussi modifier l'environnement tissulaire, en diminuant les réactions immunitaires et le rejet ou en favorisant l'intégration en augmentant l'angiogénèse. Ces molécules aussi peuvent même servir à contrôler l'expression d'un gène présent dans une cellule génétiquement modifiée, et qui est sous contrôle d'un promoteur répondant à ces molécules.

L'invention a donc aussi pour objet l'utilisation de microparticules à base d'un matériau biocompatible et biodégradable pour la préparation d'une composition pharmaceutique destinée à la réparation tissulaire ou au transfert de gène, dans laquelle lesdites microparticules comportent à leur surface des cellules d'intérêt et comprennent des molécules d'au moins une substance active sur lesdites cellules ou leur environnement lors de l'implantation des microparticules, lesdites molécules étant libérées par les microparticules de façon prolongée et contrôlée.

D'autres avantages et caractéristiques de l'invention apparaîtront des exemples qui suivent concernant la préparation et l'utilisation des MPA dans le domaine de la neurotransplantation, où il sera fait référence aux dessins en annexe dans lesquels :
- La figure 1 est une représentation schématique des MPA de l'invention.
- La figure 2 montre des cellules PC12 adhérées aux MPA, observées par microscopie optique (A) ou par microscopie électronique à balayage (B).
- La figure 3 est un histogramme représentant le comportement rotatoire induit par amphétamines des différents groupes de rats avant et après implantation de : cellules PC12 avec MPA (libérant donc du NGF), cellules PC12 avec microparticules blanches (ne libérant pas de NGF), cellules PC12 seules ou après injection de seulement le milieu de culture.
- La figure 4 est une représentation schématique de MPA pour une vaccination anti-tumorale selon l'invention.

### Exemple 1 : Présentation générale des MPA pour les greffes de cellules.

Les exemples ci-après se rapportent au domaine de la neurotransplantation (greffes de cellules nerveuses dans le système nerveux central). Des MPA biodégradables et biocompatibles d'un diamètre de 60 *µ*m ont été préparés. Ils sont constitués de PLGA et sont enrobées d'une fine couche des molécules d'adhérence synthétiques (poly-D-lysine et « fibronectin-like ») et libèrent un facteur neurotrophique, le NGF, de manière continue (pendant au moins 15 jours). Des cellules répondant au NGF comme la lignée cellulaire PC12, capables de secréter de la dopamine et de se différencier en neurones « sympathiques-like » sous l'effet de ce facteur ont été utilisées. L'efficacité de ces MPA a été évaluée avec succès, *in vivo*, en utilisant un modèle animal de la maladie de Parkinson.

Les neurotransplantations ont débuté en clinique dans les années 80, essentiellement dans le cadre de la maladie de Parkinson (Menei et al., 1991a ; 1991b). Elles se poursuivent actuellement sous la forme de recherches cliniques et restent essentiellement limitées par la disponibilité des cellules à greffer (cellules embryonnaires) et le faible taux de survie de ces cellules après implantation (5 à 10%). Des études récentes montrent que la plupart des neurones meurent dans la première semaine après transplantation, probablement à cause d'un manque de support trophique, de connexions neuronales et d'une vascularisation limitée (Emgard et al., 1999 ; Mahalik et al., 1994 ; Zawada et al., 1998). Il a été démontré que les facteurs neurotrophiques administrés en parallèle avec les cellules greffées améliorent leur survie, ils agissent aussi sur leur différenciation et le développement des synapses, aidant ainsi à leur meilleure intégration (Mahoney et al., 1999 ; Sautter et al., 1998 ; Yurek et al., 1996). Ces facteurs peuvent aussi agir de façon bénéfique sur l'environnement des cellules greffées, en modifiant la réaction inflammatoire et cellulaire (Wei et al., 1999).

### 1) Choix du facteur de croissance.

Le NGF a été choisi dans un premier temps, parce que c'est un facteur trophique qui présente plusieurs intérêts dans le cadre des neurotransplantions. Outre son action neurotrophique, il peut protéger de façon non spécifique contre l'excitotoxicité responsable d'une mortalité précoce des cellules greffées (Strijbos et Rothwell, 1995 ; Carlson et al., 1999). Le NGF présente de plus un intérêt potentiel dans le cadre des rejets de greffe. Il diminue l'expression, sur les cellules microgliales, des molécules immunologiques essentielles pour l'activation de cellules T dans le système nerveux central (Neumann et al., 1998 ; Wei et Jonakait, 1999, Aloisi et al., 2000) permettant ainsi d'empêcher le rejet ou d'établir une « immunotolérance » locale.

### 2) Choix des cellules.

Les cellules chromaffines de la médullo-surrénale ont été classiquement utilisées pour les greffes de cellules dans le cadre de la maladie de Parkinson. Par conséquent, des cellules de la lignée PC12, qui sont de assez facile à cultiver ont été utilisées. En effet, ces cellules synthétisent et libèrent de la dopamine. De plus, sous l'action du NGF, elles arrêtent de proliférer et se différencient en neurones « sympathiques-like ». Les cellules PC12 constituent aussi un bon modèle pour déterminer les conditions de production de « microcarriers », car elles ne présentent pas de propriétés d'adhérence naturelles et n'adhèrent que sur des surfaces recouvertes de molécules promouvant l'adhérence.

Le développement clinique des greffes de cellules embryonnaires reste limité par des problèmes éthiques. Il faut donc, d'ores et déjà, envisager d'autres sources cellulaires comme les cellules souches nerveuses ou les cellules souches mésenchymales de la moelle osseuse (CSM). Ces dernières sont facilement prélevables chez l'homme, peuvent se différencier *in vitro,* suivant les conditions de culture, en ostéoblastes, chondrocytes, myocites, adipocytes ou cellules souches nerveuses. Ainsi, les CSM différenciées en cellules souches nerveuses par le basic FGF expriment le récepteur à haute affinité du NGF (trkA) pourraient être utilisées dans le cade de la présente invention.

### 3) Choix des MPA.

La taille optimale dans cette application est de l'ordre de 60 *µ*m. En effet, une taille inférieure présente une surface insuffisante pour permettre l'adhérence d'un nombre acceptable de cellules. Cependant, les microsphères ne doivent pas avoir un diamètre trop important afin de pouvoir être résorbées sans trop de difficultés et être administrées facilement au travers une aiguille.

Pour l'adhésion cellulaire, les microparticules de PLGA on été enrobées avec de la « fibronectin-like » et de la poly-D-lysine. L'emploi de molécules synthétiques et non d'origine animale est indispensable pour pouvoir envisager une application clinique future. Ces microsphères enrobées permettent *in vitro* une libération continue de NGF pendant au moins quinze jours. Pour étudier in *vivo* l'effet des « microcarriers » pharmacologiquement actifs, ils ont été implantés dans le striatum de rat, après dénervation dopaminergiques totale par un neurotoxique (rat Parkinsonien). Après implantation, les cellules PC12 restent adhérées aux microparticules blanches (sans produit actif) ou libérant du NGF. Sur des animaux traités avec des « microcarriers » libérant du NGF, les PC12 se sont différenciées et présentaient des prolongements probablement en réponse à la libération du NGF. Cette différenciation s'est accompagnée d'effets comportementaux (test de rotation).

Les cellules PC12 n'ont été utilisées dans cette étude que pour mettre au point les MPA, mais d'autres cellules peuvent être employées. Les greffes de cellules d'origine embryonnaire ont été très étudiées dans des modèles animaux de maladies neurodégénératives. Ces études ont d'ailleurs mené à la réalisation d'essais clinique dans la maladie de Parkinson et la Chorée de Huntington. Cependant, les effets modestes de ces greffes, rapportés à une forte mortalité des cellules greffées conduisent à repenser la méthodologie (Lindvall, 1997). Dans le cadre de la maladie de Parkinson et des greffes de neurones embryonnaires dopaminergiques, des études ont montré que l'exposition de ces cellules au GDNF (Tornqvist et al. 2000), ou encore l'utilisation de « microcarriers » (Saporta et al 1997) augmentent leur survie. Des microparticules libérant du GDNF peuvent aussi être préparées pour favoriser la greffe de neurones dopaminergiques embryonnaires et étudier leur efficacité chez le rat.

### Exemple 2 : Préparation des MPA PC12/NGF.

### 1) Préparation des microparticules.

Les microparticules sont obtenues par le procédé de double émulsion (H/L/H) évaporation/extraction de solvant.

La phase aqueuse est constituée de 60 *µ*l de tampon citrate (16mM, pH6), 2,5 mg de HSA, (ou autre molécule ayant un pouvoir tensioactif) 90*µ*l de PEG 400 et 100 *µ*g de NGF. 50 mg - 150 mg de PLGA 37,5/25 (Poly D,L-lactide-co-glycolide, Mw 21 000 Da, I=1,7) ou un autre polymère biodégradable et biocompatible, sont dissous dans 2 ml d'une solution organique constituée de 75% de dichlorométhane et de 25% acétone. L'émulsion primaire est réalisée à partir de ces deux phases par sonication à 0-4°C (15 s, 5-6 W). Cette émulsion primaire est ajoutée sous agitation mécanique (200 tpm) à 30-150 ml d'une solution d'alcool polyvinylique (0,8-4,5%, 4-8°C) contenant 10% (P/V) de NaCl, contenant de 0 à 2% de dichlorométhane. L'agitation est maintenue de 1 à 7 min, l'émulsion secondaire est ensuite versée dans 400 ml d'une solution aqueuse à 10% de NaCl sous agitation magnétique (25-50 min). La phase aqueuse d'extraction peut-être ajoutée en partie à l'émulsion secondaire. Les microparticules sont ensuite filtrées (0,45 *µ*m, HVLP, Millipore), lavées par 5 fois 100 ml d'eau distillée puis lyophilisées et conservées à +4°C.

Le rendement d'encapsulation est de l'ordre de 85%, mais selon les conditions de fabrication il peut atteindre 97% (Péan et al 1998).

### 2) Enrobage des microparticules.

L'enrobage des microparticules par une association de « fibronectin-like » (16 *µ*g/mL) avec de la poly-D-lysine (12 *µ*g/mL) constitue la condition permettant d'avoir une adhérence et une différenciation optimale des cellules PC12.

Les conditions d'enrobage des microparticules (vitesse et temps d'agitation) ont été mises au point par agitation de la suspension de microsphères en présence de « fibronectin-like » et de poly-D-lysine. Après différents essais, nous avons choisi une vitesse d'agitation de 15 tours par minute (tpm) et une durée d'enrobage de 2 heures à 37°C.

### 3) Adhésion des cellules sur les microparticules.

Pour mettre au point les conditions d'adhérence des cellules sur les « microcarriers », différentes vitesses et temps d'agitation des cellules en présence des microparticules ont été testés. L'adhérence des cellules PC12 a donc été maximale pour une agitation à 3 tpm pendant 4 heures à 37°C dans un incubateur à 5% CO₂.

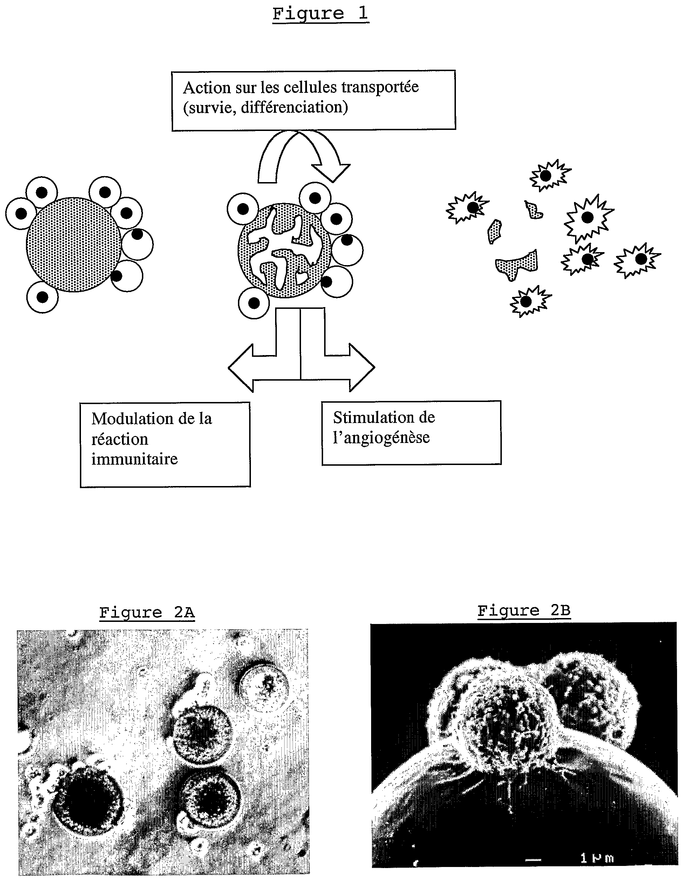

Les tests de séparation des cellules seules des cellules adhérées aux microparticules ont conduit à centrifuger la suspension microparticules/cellules à 135 g pendant 10 secondes. Ce traitement, ainsi que le passage du culot dans une seringue Hamilton 10 *µ*L, utilisée pour les greffes, ne produit pas une diminution du nombre de microparticules présentant des cellules à leur surface. Dans ces conditions, et après comptage au microscope de la suspension microparticules/cellules il a été possible d'obtenir environ 90% des microparticules avec des cellules adhérées à leur surface. En moyenne, on trouve 8 à 10 cellules par « microcarrier ». Par contre, lorsque les microsphères ne sont pas enrobées, seulement 5% d'entre elles possèdent des cellules collées à leur surface. L'enrobage des microparticules par une association de fibronectin-like et de poly-D-lysine est donc essentiel pour que les cellules y adhèrent. Cette adhérence a été confirmée par des images de microscopie électronique à balayage (figure 2).

### 4) Caractérisation in vitro

L'enrobage a été caractérisé par microscopie de force atomique et montre que cet enrobage se dépose de façon homogène sur la particule et diminue la porosité de sa surface. Dans des conditions d'enrobage citées précédemment l'épaisseur de l'enrobage est de 20nm. Après lyophilization l'enrobage reste intact et maintient son efficacité d'adhésion et différenciation dans un test d'adhésion in vitro.

Dans les conditions d'adhésion citées précédemment lorsque 1.5x10⁵ cellules sont mises en présence des MPA, 5x10⁴ cellules en moyenne adhérent sur chaque MPA.

### Exemple 3 : Résultats des MPA NGF/PC12 sur modèles animaux.

Les MPA libèrent du NGF de façon prolongée et contrôlée. En effet, les premiers résultats de la cinétique de libération *in vitro,* montrent que pour 200 µg de NGF encapsulé, 15% sont libérés de façon continue pendant les deux premières semaines. L'implantation de 0,5 mg de MPA conduirait ainsi à la libération de 5-10 ng de NGF par jour, ce qui est en accord avec les quantités nécessaires à une action de NGF sur les cellules.

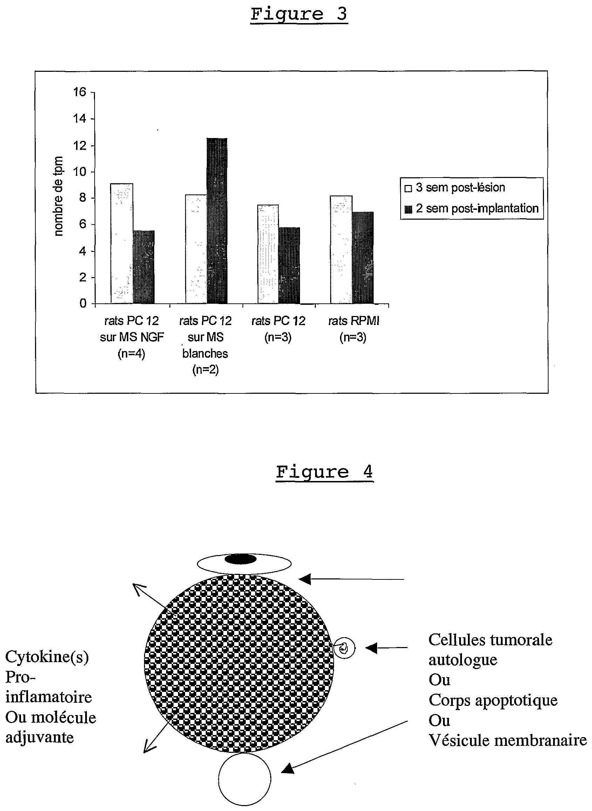

Après implantation dans le striatum dénervé de « rats Parkinsoniens », les cellules PC12 restent bien adhérées sur les microparticules. Les cellules transportées expriment toujours la tyrosine hydroxylase et sont donc capables de produire de la dopamine. Deux semaines après implantation, les microparticules ne sont pas toujours dégradées et servent encore de support aux cellules. En effet, elles sont encore sphériques et présentent un aspect assez lisse, sans pores de vacuolisation. De façon générale, les cellules adhérées aux microparticules, avec ou sans NGF, ont un aspect différencié par rapport aux cellules greffées seules dans le striatum. En observant ces cellules à fort grossissement, on remarque que sur les microparticules libérant du NGF, les prolongements sont plus longs faisant à peu près 2 à 3 fois la taille du corps cellulaire (figure 2B). Au niveau comportemental, les premiers résultats des tests de rotation induits par amphétamine, montrent que seuls les rats ayant reçu les cellules avec les microparticules libérant du NGF se sont améliorés (figure 3). Parmi les résultats, il semble que les cellules PC12 adhérées aux microparticules ne libérant pas de NGF arrêtent de proliférer.

### Résultats sur modèles animaux

Un immunomarquage grâce à un anticorps reconnaissant le site actif du NGF deux semaines après implantation des microsphères dans le striatum du rat « Parkinsonien » montre que le NGF est bien libéré sous une forme biologiquement active. A deux semaines, du NGF est encore détecté dans les MPA et libéré tout autour de façon homogène et sur une distance d'au moins 40 µm.
Dans certains endroits autour de MPA libérant du NGF des réseaux des neurites sont aussi observés, démontrant bien la libération du facteur de croissance qui ainsi stimule la différenciation de cellules PC12.

Les cellules PC12, comme d'autres lignées cellulaires ou même des cellules souches, peuvent former des tumeurs après implantation Des marqueurs de prolifération montrent que dans les greffes de cellules PC12 adhérées aux MPA il y une diminution de nombre des cellules qui prolifèrent. Cet effet est plus marqué avec les MPA libérant du NGF.

La mort des cellules par apoptose est aussi diminué dans le greffe des cellules PC12 adhérés aux MPA libérant ou non de NGF.

Au niveau comportemental, le test de rotations induites par amphétamine, montrent que seuls les rats ayant reçu les cellules avec les MPA libérant du NGF se sont améliorés confirmant l'efficacité des MPA dans ce modèle.

### Exemple 4 Préparation des MPA libérant du GDNF transportant des Cellules embryonnaires dopaminergiques (E-dopa)

Après incubation des MPA enrobés de la poly-D-lysine avec des cellules à un cycle de rotation de 6 tours par minute pendant au minimum une heure, des cellules E-dopa adhèrent à la surface de 70% à 90% des MPA. Le nombre des cellules adhérées est assez variable, aux alentours de 5-30 cellules par MPA.

### Exemple 5 :Présentation générale des MPA pour la vaccination anti-tumorale (non revendiqué)

Notre expérience clinique en vaccination anti-tumorale ainsi que les résultats publiés dans la littérature confirment que la culture des cellules tumorales est une étape limitante dans ce type d'approche. Le rendement est faible et le délai pour obtenir le nombre de cellule nécessaire est souvent incompatible avec la rapidité d'évolution de la tumeur. Les cellules autologue restant actuellement la source la plus appropriée d'Antigènes spécifiques de tumeur (AST), que ce soit pour le chargement de cellules dendritiques in vitro, ou la vaccination sous-cutanée, il est utile de développer un système pouvant être préparé rapidement, à partir de peu de cellules, capable de présenter la majorité des antigènes tumoraux ainsi qu'un adjuvant aux cellules dendritiques.

Nous formulons pour cela particules bio-artificielles reposant sur le concept de microcarrier pharmacologiquement actifs (MPA), développé dans l'INSERM ERIT-M 0104. Il s'agit de microsphères de 5 à 30*µ*m (environ) de diamètre, constituées d'un co-polymère biocompatible et biodégradable (comme le poly[acide lactique-co-acide glycolique] ou PLGA), pouvant libérer de façon prolongée et contrôlée un adjuvant comme une cytokine pro-inflammatoire (comme le GM-CSF, l'IL2, l'IL12 ou l'IL18). Par leurs propriétés de surface (pelliculage par des molécules d'adhésion cellulaire synthétiques), ces microparticules peuvent porter à leur surface des antigènes tumoraux préparés à partir de cellules tumorales autologues (vésicules membranaires, corps apoptotiques, exosomes, extrait protéique, ARNm, ADN).

Ces MPA portant des antigènes tumoraux à leur surface et libérant de façon contrôlée un adjuvant, comme une cytokine est destiné à :
- être mis en contact avec des cellules dendritiques in vitro (dans le cadre de vaccination avec des cellules dendritiques issues de cellules souches sanguines)
- ou à être administrés au patient directement, par voie sous-cutanée, intradermique ou intramusculaire.

Le caractère immunogène du MPA est dû non seulement à l'association de l'antigène tumoral et de la libération prolongée de cytokine, mais aussi à la présentation de l'antigène sur un système microparticulaire. C'est un mode de présentation idéal pour la reconnaissance de l'antigène par les cellules dendritiques. Cette stimulation immunitaire non spécifique, ou le rôle adjuvant des microparticules, est démontrée depuis longtemps (Mescher et Rogers, 1996, Mesher et Savelieva, 1997 ;Nakaoka et al., 1995, Rogers and Mescher 1992, Scheicher et al. 1995a, 1995b, Venkataprasad et al., 1999).

Les microsphères de PLGA ont été déjà décrites comme pouvant libérer des cytokines immunostimulantes et/ou anti-tumorales (Golumbek et al, 1993 ; Mullerad et al. 2000, Pettit et al., 1997), et nous avons déjà montré notre capacité dans le laboratoire à développer des microsphères libérant des agents anti-tumoraux ou des protéines recombinantes (Menei et al 1996, 1999, 2000 ; Péan et al., 2000).

### Exemple 6 : MPA porteurs de membranes plasmatiques dans la vaccination anti-gliome (non revendiqué)

Les microsphères de PLGA utilisées, (sans et avec une cytokine activatrice, le GM-CSF) ont été fabriquées par technique d'évaporation de solvant comme précédemment décrit (Menei et al., 1993 , Menei et al. 96, Pean et al, 2000). Une filtration, a permis de sélectionner les microsphères de petites tailles (5 à 30 *µ*m de diamètre).

La purification des membranes plasmiques des cellules GS-9L a été réalisée en incubant 1,5.10⁸ cellules 15 minutes à 4°C dans un tampon hypotonique (KCl 42 mM, Hépes 10 mM pH7, MgCl₂ 4,5 mM et 1% d'inhibiteurs de protéases) en effectuant environ 50 passages dans une seringue de 30 gauge. Les cellules ainsi rompues ont alors été centrifugées (250 g ; 10 minutes ; 4°C) pour séparer les membranes dans le surnageant, des cellules non lysées et des noyaux rassemblés en un culot. Les membranes ont été alors récupérées après une étape d'ultracentrifugation (100 000 g, 90 minutes, 4°C). Elles ont ensuite été placées dans un système biphasique poly-éthylène-glycol (PEG 8000) / Dextran T500 équilibré 48 heures à l'avance, pour être alors centrifugées (3 000 g, 15 minutes, 4°C)). Les membranes plasmiques sont alors disposées à l'interphase du système biphasique, par affinité pour les deux phases. Après récupération, 2 lavages dans un tampon de sucrose 0,25 M, Tris HCl 1 M ont été effectués (100 000g, 30 minutes, 4°C). Les membranes ont été conservées dans ce même tampon à -80°C jusqu'à leur utilisation.). Parmi les protocoles d'adsorption et de formulation testés, celui qui semble être le plus efficace met en présence les membranes et les microsphères sans enrobage préalable dans du tampon Tris pH 6,8. D'autres protocoles d'adsorption optimum pour les fractions protéiques, vésicules membranaires, corps apoptotiques et exosomes sont en cour d'élaboration. Ces protocoles sont validés par radiomarquage des fractions protéiques par l'I¹²⁵, par immunofluorescence directe de marqueurs membranaires en microscopie confocale puis par l'analyse de l'activité enzymatique des structures adsorbées

(non revendiqué) Exemple d'adsorption des membranes plasmiques de cellules GS-9L sur les microsphères de PLGA : La préparation de membranes plasmiques purifiées a été soniquée pendant 30 secondes pour réduire la taille des agrégats de vésicules membranaires. L'équivalent de 10 *µ*g de protéines membranaires ont alors été mises en présence de 20 000 microsphères de PLGA. L'ensemble a été placé sous rotation (60 tpm) toute la nuit à 4°C.
Afin de déterminer la quantité de protéines membranaires récupérées, un dosage appliquant le test de Lowry a été effectué En utilisant une gamme étalon de BSA (comprise entre 0,2 et 1 *µ*g/*µ*L), la concentration protéique des échantillons a pu être calculée. L'aliquote, appelée aliquote initiale (Ai), prélevée en début de purification (juste après la lyse) et contenant l'ensemble des protéines de la cellule, présentait une concentration protéique entre 7000 et 10 000 *µ*g/mL. En revanche, en fin d'enrichissement, on obtient une concentration protéique comprise entre 200 et 300 *µ*g/mL. Nous avons donc récupéré 3 % des protéines totales.

Le profil des protéines isolées a été analysé par migration sur gel de polyacrylamide montrant un profil de migration intermédiaire des profils des fractions soluble et non soluble des lysats cellulaires. C'est la fraction non soluble qui contient majoritairement des protéines membranaires. Le dosage de l'activité spécifique de la 5'-nucléotidase. Et de Fas-liguant par Western blot va dans le sens d'un enrichissement de ces protéines membranaires, prouvant la nature membranaire du matériel enrichi. L'immunofluorescence directe de marqueurs membranaires en microscopie confocale (5'-nucléotidase l'intégrine, ICAM-1) confirme la fixation des protéines membranaires sur la microparticule

### REFERENCES BIBLIOGRAPHIQUES

- Aloisi F., Ria F., Adorini L. Régulation of T-cell responses by CNS-presenting cells : différent for microglia and astrocytes. Immunol Today, 2000, 21 : 141-147.
- Benoit J.P., Faisant N., Venier-Julienne M.C., Menei P. Development of microspheres for neurological disorders : from basics to clinical applications. J Control Release, 1999, 65 : 285-296
- Borlongan C.V., Saporta S., Sanberg P.R. Intrastriatal transplantation of rat adrenal chromaffin cells seeded on microcarrier beads promote long-term functional recovery in hemiparkinsonian rats. Exp Neurol., 1998, 151 : 203-214.
- Carlson N.G., Wieggel W.A., Chen J., Bacchi A., Rogers S.W., Gahring L.C. Inflammatory cytokines IL-la, IL-1β, IL-6 and TNF-α impart neuroprotection to an excitotoxin through distinct pathways. J Immunol., 1999, 163 : 3963-3968.
- Cherskey B.D., Sapirstein V.S., Geraci A.L. Adrenal chromaffin cells on microcarriers exhibit long-term functional effects when implanted into the mammalian brain. Neurosci., 1996, 75 : 657-664.
- Date I., Ohmoto T. Neural transplantation and trophic factors in parkinson's disease : spécial reference to chromaffin cell grafting, NGF support from pretransected peripheral nerve, and encapsulated dopamine-secreting cell grafting. Exp. Neurol., 1996, 137 : 333-344.
- Dehaut F., Montero-Menei C.N., Alhayek G. and Pouplard-Barthelaix A. Differential behavior of PC12 cells grafted into the rat hippocampus and striatum. Neurosci. Letters, 1993, 153 : 41-44.
- Demetriou A.A., Levenson S.M., Novikoff P.M., Novikoff A.B., Roy Chodhury N., Whitting J., Reisner A., Roy Chowdury J. Survival organization and function of microcarrier-attached hepatocytes transplanted in rats. Proc. Natl. Acad. Sci. USA, 1986, 83 : 7475-7479.
- Emgard M., Karlsson J., Hansson O., Brundin P. Patterns of cell death and dopaminergic neuron survival in intrastriatal nigral grafts. Exp. Neurol., 1999, 160 : 279-288.
- Golumbek PT, Azhari R, Jafee EM, Levitsky HI, Lazenby A, Leong K, Pardoll DM Controlled release, biodégradable cytokine depot ; a new approach in cancer vaccine design. Cancer Res 1993, 53 :5841-5844
- Junard E.D., Montero C.N., Hefti F. Long-term administration of mouse nerve growth factor to adult rats with partial lesions of the cholinergic septo-hippocampal pathway. Exp. Neurol., 1990, 110(1) : 25.
- Kneser U., Kaufmann P.M., Fiegel H.C., Pollok J.M., Kluth D., Herbst H. Interactions of hepatocytes and pancreatic islets cotransplanted in polymeric matrices. Virchows Arch., 1999, 435 : 125-132.
- Lindvall O.Neural transplantation : a hope for patients with Parkinson's disease. Neuroreport., 1997, 8 : iii-x.
- Mahalik T.J. Hahn W.E., Clayton G.H., Owens G.P. Programmed cell death in developing grafts of fetal substantia nigra. Exp. Neurol., 1994, 129 : 27-36.
- Mahoney M.J., Saltzman W.M. Millimeter-scale positioning of a nerve growth factor source and biological activity in the brain. Proc. Natl. Acad. Sci. USA, 1999, 96 : 4536-4539.
- Menei P., Wion D., Pouplard-Barthelaix A., Brachet P., Fournier D., Alhayek G., Mercier P., Guy G. Greffe intra-cérébrale d'une lignée de cellules chromaffines: aspects immunologiques et rôle du Nerve Growth Factor dans la survie et la différenciation du greffon. Neurochirurgie, 1989, 35 : 158-163.
- Menei P., Pouplard-Barthelaix A., Guy G. Complications et risques biologiques des greffes neuronales. Neurochirurgie, 1991a, 37 : 364-376.
- Menei P., Guy G., Pouplard-Barthelaix A. Mise au point : Les greffes intra-cérébrales dans la maladie de Parkinson. La Presse Médicale, 1991b, 11 : 513-517.
- Menei P., Daniel V., Montero-Menei C.N., Brouillard M., Pouplard-Barthelaix A., Benoit J.P. Biodégradation and brain tissue reaction to poly(d,l-lactide-co-glycolide) microspheres. Biomaterials, 1993, 14 : 470-478.
- Menei P., Boisdron-Celle M., Croue A., Guy G., Benoit J.P. Drug targeting by stereotactic implantation of biodegradable microspheres.Neurosurgery, 1994a, 34 : 1058-1064.
- Menei P., Croue A., Daniel V., Pouplard-Barthelaix A., Benoit J.P. Fate and brain biocompatibility of three types of microspheres implanted into the brain. J. Biomed. Mater. Res., 1994b, 28 : 1079-1085.
- Menei P., Venier-Julienne M.C., Benoit J.P. Drug delivery into the brain using polymeric devices S.T.P. PharmaSciences, 1997, 7: 53-61.
- Menei P., Montero-Menei C., Whittemore S., Bunge R.P., Bunge M.B. Schwann cells genetically modified to secrete human BDNF promote enhanced axonal regrowth across transected adult rat spinal cord. Eur J Neurosci., 1998, 10 : 607-621.
- Menei P., Venier-Julienne M.C., Gamelin E., Saint-Andre J.P., Hayek G., Jadaud E., Fournier D., Mercier P., Guy G., Benoit J.P. Local and sustained delivery of 5-fluorouracil from biodegradable microspheres for the radiosensitization of glioblastoma : A pilot study. Cancer, 1999, 86/2 : 325-333.
- Menei P., Pean J.M., Nerrière-Daguin V., Jollivet C., Brachet P., Benoit J.P. Intrastriatal NGF-releasing biodegradable microspheres prevent the degeneration of cholinergic and non cholinergic striatal neurons in a model of Huntington disease. Exp Neurol., 2000, 161 : 259-272.
- Mescher MF, Rogers JD Immunotherapy of established murine tumors with large multivalent immunogen and cyclophosphamide. J Immunother Emphasis Tumor Immunol, 1996, 19 : 102-112
- Mescher MF, Savelieva E Stimulation of tumor-specific immunity using tumor cell plasma membrane antigen Methods, 1997, 12 : 155-164
- Montero C.N., Hefti F. Rescue of lesioned septal cholinergic neurons by nerve growth factor : specificity and requirement for chronic treatment J. Neurosci., 1988, 8(8) : 2986-2999.
- Montero C.N., Hefti, F. Intraventricular administration of nerve growth factor into aged rats. Neurobiol of Aging,. 1989, 10: 739-743.
- Montero-Menei C.N., Pouplard-Barthelaix A., Gumpel M., Baron A. Pure Schwann cell suspension grafts promote regeneration of the lesioned septo-hippocampal cholinergic pathway. Brain Research., 1992, 570 : 198-208.
- Mullerad J, Cohen S, Voronov E, Apte RN Macrophage activation for the production of immunostimulatory cytokines by delivering interleukin 1 via biodegradable microspheres. Cytokine, 2000, 12 : 1683-1690
- Nakaoka R, Tabata Y, Ykada Y Potentiality of gelatin microspheres as immunological adjuvant. Vaccine, 1995, 13 : 633-661
- Neumann H., Misgeld T., Matsumuro K., Wekerle H. Neurotrophins inhibit major histocompatibility class II inducibility of microglia : involvement of the p75 neurotrophin receptor. Proc. Natl. Acad. Sci. USA, 1998, 95 : 5779-5784.
- Pean J. M., Venier-Julienne M. C., Boury F., Menei P., Denizot B., and Benoit J. P. (1998) NGF release from poly(d,l-lactide-co-glycolide) microspheres. Effects of some formulation parameters on encapsulated NGF stability. J. Control Release. 56, 175-187.
- Péan J.M., Boury F., Venier-Julienne M.C., Menei P., Proust JE, Benoit J.P. Why does PEG400 co-encapsulation improve NGF stability and release from PLGA biodégradable microspheres? Pharmaceutical Res., 1999, 16 : 1294-1299.
- Péan J.M, Menei P., Morel O.,. Montero-Menei C.N., Benoit J.P. Intraseptal implantation of NGF releasing-microspheres promote the long term survival of axotomized cholinergic neurons. Biomaterials, 2000, 21 : 2097-2101.
- Pettit DK, Lawter JR, Huang WJ, Pankey SC, Nightlinger NS, Lynch DH, Schuh JA, Morrissey PJ, Gombotz WR Characterization of poly(glycoloiude-co-D,L,lactide) :poly(D,L, lactide) microspheres for controlled release of GM-CSF. Pharm Res, 1997, 14 : 1422-1430.
- Rogers J, Mescher MF Augmentation of in vivo cytotoxic T lymphocyte activity and réduction of tumor growth by large multivalent immunogen. J Immunol, 1992, 149 : 169-276.
- Saporta S., Borlongan C., Moore J., Mejia-Millan E., Jones S.L., Bonness P., Randall T.S., Allen R.C., Freeman T.B., Sanrerg P.R. Microcarrier enhanced survival of human and rat fetal ventral mesencephalon cells implanted in the rat striatum. Cell Transpl., 1997, 6 : 579-584.
- Sautter J., Tseng J.L., Braguglia D. et al. Implants of polymer-encapsulated genetically modified cells releasing glial cel line-derived neurotrphic factor improve survival , growth and function of fetal dopaminergic grafts. Exp Neurol., 1998, 149 : 230-236.
- Scheicher C, Mehling M, Dienes HP, Reske K Uptake of bead-adsorbed versus soluble antigen by bone marrow derived dendritic cells trigers their activation and increase their antigen presentation capacity. Adv Exp Med Biol 1995, 378 : 253-255
- Scheicher C, Mehling M, Dienes HP, Reske K Uptake of microparticle-adsorbed protein antigen by bone marrow derived dendritic cells results in up regulation of IL1 alpha and IL12 p40 :P35 and triggers prolonged, efficient antigen presentation. Eur J Immunol, 1995, 25 : 1566-1572
- Strijbos P.J., Rothwell N.J. Interleukin-1β attenuates excitatory amino-acid-induced neurodegeneration in vitro : involvement of nerve growth factor. J. Neurosci., 1995, 15 : 3468.
- Te Velde A.A., Bosman, D.K., Oldenburg J., Sala M., Maas M.A., Chamuleau R.A. Three different hepatocytes transplantation techniques for enzyme deficiency disease and acute hepatic failure. Artif. Organs, 1992, 16 : 522-526.
- Törnqvist N., Björklund L., Almqvist P., Wahlberg L. and Strömberg I. Implantation of bioactive growth factor-secreting rods enhances fetal dopaminergic graft survival, outgrowth density, and functional recovery in a rat model of Parkinson's disease. Exp. Neurol., 2000, 164 : 130-138.
- Varani J., Inman DR., Fliegiel SE. And Hillegas WJ. Use of recombinant and synthetic peptides as attachment factors for cells on microcarriers. Cytotechnology, 1993, 13 : 89-98.
- Venkataprasad N, Coombes AG, Singh M, Rohde M, Wilkinson K, Hudecz F, davis SS, Vordermeier HM Induction of cellular immunity to a mycobactérial antigen adsorbedon lamemmar particles of lactides polymers. Vaccine, 1999, 17 : 1814-1819
- Vezier J., Lesourd M., Jollivet C., Montero-Menei C., Benoit J.P., Menei P. Brain biocompatibility of drug releasing biodegradable microspheres analyzed by scanning and electronic microscopy.(soumis).
- Voigt M., Schauer M., Schaefer D.J., Andree C., Horch R., Stark G.B.Cultured epidermal keratinocytes on a microspherical transport system are feasible to reconstitute the epidermis in full-thickness wounds. Tissue Eng., 1999, 6 : 563-572.
- Wei R., Miller Jonakait G. Neurotrophins and the anti-inflammatory agents interleukin-4 (IL-4), IL-10, IL-11 and transforming growth factor-b1 (TGF-β1) down-regulate T cell costimulatory molecules B7 and CD40 on cultured rat microglia. J. Neuroimmunol., 1999, 95 : 8-18.
- Wood KJ. And Prior TG. Gene therapy in transplantation. Curr. Opin. Mol. Ther., 2001, 3(4) : 390-398.
- Yurek D.M., Lu W., Hipsen S., Wiegand S.J. BDNF enhances the functional reinnervation of the striatum by grafted fetal dopamine neurons. Exp. Neurol., 1996, 137 : 105-118.
- Zawada W.M., Zastrow D., Clarkson E.D., Adams F., Bell K.P., Freed C.R. Growth factors improve immediate survival of embryonic dopamine neurons after transplantation into rats. Brain Res., 1998, 786 : 96-103.

## Revendications

1. Microparticule à base d'un matériau biocompatible et biodégradable, ladite particule comporte à sa surface des cellules d'intérêt choisies parmi des cellules embryonnaires ou des cellules souches et comprend des molécules d'au moins une substance active sur lesdites cellules ou leur environnement lors de l'implantation des microparticules choisie parmi les facteurs de croissance, les cytokines, les immunomodulateurs ou facteurs agissant sur la différenciation cellulaire, lesdites molécules étant, libérées par les microparticules de façon prolongée et contrôlée,
ladite microparticule est enrobée d'un composé permettant l'adhésion des cellules, ledit composé étant en outre éventuellement actif sur lesdites cellules, choisi parmi la poly-D-lysine, la poly-L-lysine, la polyornithine, la polyéthylèneamine, ou molécule synthétique ou non appartenant à la matrice extracellulaire choisie parmi la fibronectin-like, des polymères synthétiques sur lesquels sont greffées des séquences RGD ou de la lysine ou un mélange de ceux-ci, et
ladite microparticule a un diamètre compris entre 10 et 500 µm, et
ledit matériau biodégradable et biocompatible est un polymère ou un copolymère de polyester.

2. Microparticule selon la revendication 1, dans laquelle le polyester est un poly (α-hydroxyacide).

3. Microparticule selon la revendication 2 dans laquelle le poly (α-hydroxyacide) est un polylactide ou un polylactide co-glycolide.

4. Microparticule selon l'une quelconque des revendications précédentes, dans laquelle les molécules incorporées dans les microparticules sont celles d'au moins une substance active choisie dans le groupe des facteurs de croissance.

5. Microparticule selon la revendication 4 dans laquelle ladite substance active est choisie dans le groupe comprenant NGF, BNDF, NT-3, les TGFβs, la famille du GDNF, les FGFs, EGF, et PDGF.

6. Microparticule selon l'une quelconque des revendications précédentes, ladite microparticule ayant un diamètre de 60 µm.

7. Microparticules selon l'une quelconque des revendications précédentes, lesdites cellules souches étant des cellules souches nerveuses ou des cellules souches mésenchymales de la moelle osseuse.

8. Microparticules selon l'une quelconque des revendications précédentes, dans laquelle lesdites molécules sont libérées par les microparticules de façon prolongée et contrôlée pendant au moins 15 jours.

9. Microparticules selon l'une quelconque des revendications précédentes pour son utilisation pour la réparation tissulaire.

## Patentansprüche

1. Mikropartikel auf Grundlage eines biokompatiblen und bioabbaubaren Materials, wobei dieser Partikel auf seiner Oberfläche Zellen von Interesse, ausgewählt aus embryonalen Zellen oder Stammzellen, und Moleküle von mindestens einer aktiven Substanz auf diesen Zellen oder in deren Umgebung bei Implantation der Mikropartikel, ausgewählt aus Wachstumsfaktoren, Zytokinen, Immunmodulatoren oder Faktoren, die auf die zelluläre Differenzierung einwirken, umfasst, wobei diese Moleküle auf verzögerte und kontrollierte Weise durch die Mikropartikel freigesetzt werden, wobei der Mikropartikel von einer Zusammensetzung überzogen ist, die die Adhäsion der Zellen erlaubt, wobei die Zusammensetzung außerdem gegebenenfalls aktiv auf diesen Zellen ist, ausgewählt aus Poly-D-Lysin, Poly-L-Lysin, Polyornithin, Polyethylenamin oder einem synthetischen oder nicht in der extrazellulären Matrix auftretendem Molekül, ausgewählt aus Fibronektin-artigen, synthetischen Polymeren, auf welchen die Sequenzen RGD auftreten, oder Lysin oder einer Mischung von beiden und
wobei der Mikropartikel einen Durchmesser von 10 bis 500 µm hat und wobei das bioabbaubare und biokompatible Material ein Polymer oder ein Copolymer eines Polyesters ist.

2. Mikropartikel nach Anspruch 1, bei welchem der Polyester eine Poly(α-hydroxysäure) ist.

3. Mikropartikel nach Anspruch 2, bei welchem das Poly(α-hydroxysäure) ein Polylactid oder ein Polyactid-Co-Glycolid ist.

4. Mikropartikel nach einem der voranstehenden Ansprüche, bei welchem die in die Mikropartikel eingebauten Moleküle solche von mindestens einer aktiven Substanz sind, ausgewählt aus der Gruppe der Wachstumsfaktoren.

5. Mikropartikel nach Anspruch 4, bei welchem diese aktive Substanz ausgewählt ist aus der Gruppe, umfassend NGF, BNDF, NT-3, TGFβs, die Familie der GDNF, FGFs, EGF und PDGF.

6. Mikropartikel nach einem der vorgenannten Ansprüche, wobei der Mikropartikel einen Durchmesser von 60 µm hat.

7. Mikropartikel nach einem der vorgenannten Ansprüche, wobei die Zellen Nervenstammzellen oder mesenchymale Stammzellen des Knochenmarks sind.

8. Mikropartikel nach einem der voranstehenden Ansprüche, bei welchen die von den Mikropartikeln freigesetzten Moleküle auf verzögerte und kontrollierte Weise während mindestens 15 Tagen freigesetzt werden.

9. Mikropartikel nach einem der voranstehenden Ansprüche für die Verwendung bei der Gewebewiederherstellung.

## Claims

1. Microparticle based on a biocompatible and biodegradable material, said particle comprises on its surface cells of interest chosen from embryonic cells or stem cells and comprises molecules of at least one active substance on said cells or their environment at the time of implantation of the microparticles chosen from growth factors, cytokines, immunomodulators or factors acting on cell differentiation, said molecules being released by the microparticles in a prolonged and controlled fashion, said microparticle is coated with a compound allowing adhesion of the cells, said compound also optionally being active on said cells, chosen from poly-D-lysine, poly-L-lysine, polyornithine, polyethylenamine, or a molecule, synthetic or not, belonging to the extracellular matrix chosen from fibronectin-like, synthetic polymers on which are grafted RGD sequences or lycine or a mixture of these, and said microparticle has a diameter of between 10 and 500 µm, and
said biodegradable and biocompatible material is a polymer or a polyester copolymer.

2. Microparticle according to claim 1, in which the polyester is poly(α-hydroxyacid).

3. Microparticle according to claim 2, in which the poly(α-hydroxyacid) is a polylactide or a poly(lactide-co-glycolide.

4. Microparticle according to any of the preceding claims, in which the molecules incorporated in the microparticles are those of at least one active substance chosen from the group of growth factors.

5. Microparticle according to claim 4, in which said active substance is chosen from the group comprising NGF, BNDF, NT-3, TGFβs, the GDNF family, FGFs, EGF and PDGF.

6. Microparticle according to any of the preceding claims, said microparticle having a diameter of 60 µm.

7. Microparticles according to any of the preceding claims, said stem cells being nerve stem cells or mesenchymal stem cells of bone marrow.

8. Microparticles according to any of the preceding claims, in which said molecules are released by the microparticles in a prolonged and controlled fashion over at least 15 days.

9. Microparticles according to any of the preceding claims, for use thereof for tissue repair.
